# EUROPÄISCHE PATENTSCHRIFT

(11) **EP 1 833 499 B1**
(45) Veröffentlichungstag und Bekanntmachung des Hinweises auf die Patenterteilung: **19.11.2008**
(21) Anmeldenummer: 05826512.5
(22) Anmeldetag: 14.12.2005
(51) Int. Cl.: A61K 36/45

(54) **HOMÖPATHISCHES MITTEL ZUR PROPHYLAXE GEGEN VON PARASITEN HERVORGERUFENEN KRANKHEITEN**
HOMEOPATHIC AGENT FOR THE PROPHYLAXIS OF DISEASES CAUSED BY PARASITES
AGENT HOMEOPATHIQUE DE PROPHYLAXIE CONTRE DES MALADIES CAUSEES PAR DES PARASITES

(30) Priorität: 18.12.2004 DE 102004061011
(43) Veröffentlichungstag der Anmeldung: 19.09.2007
(73) Patentinhaber: Weineck, Andrea, 69118 Heidelberg/Zgh (DE)
(72) Erfinder: Weineck, Andrea, 69118 Heidelberg/Zgh (DE)
(74) Vertreter: Kesselhut, Wolf
(86) Internationale Anmeldenummer: PCT/EP2005/013442
(87) Internationale Veröffentlichungsnummer: WO 2006/063815

(56) Entgegenhaltungen:
- DATABASE BIOSIS [Online] BIOSCIENCES INFORMATION SERVICE, PHILADELPHIA, PA, US; 1988, THORSELL W: "INTRODUCTORY STUDIES OF PLANT EXTRACTS WITH MOSQUITO REPELLING PROPERTIES" XP002372026 Database accession no. PREV198988036449 & FAUNA OCH FLORA NATURHISTORISKA RIKSMUSEET, Bd. 83, Nr. 5, 1988, Seiten 202-207, ISSN: 0014-8903
- DATABASE BIOSIS [Online] BIOSCIENCES INFORMATION SERVICE, PHILADELPHIA, PA, US; 1979, KRYLOVA I L ET AL: "EFFECT OF ECOLOGICAL FACTORS ON THE CONTENT OF ESSENTIAL OIL AND TANNINS IN THE LEAVES OF LEDUM-PALUSTRE" XP002372027 Database accession no. PREV198171040361 & RASTITEL'NYE RESURSY, Bd. 15, Nr. 4, 1979, Seiten 575-583, ISSN: 0033-9946

## Beschreibung

Die vorliegende Erfindung betrifft ein homöopathisches Mittel, enthaltend
a) Ledum Pallustre in Form einer homöopathischen Verdünnung in einem inerten Trägermaterial und
b) wenigstens ein Sekret eines Parasiten oder eines durch Parasiten Erkrankten oder auch eines Zwischenwirts in Form einer homöopathischen Verdünnung in einem inerten Trägermaterial.

Weiterhin betrifft die vorliegende Erfindung ein Verfahren zur Herstellung des erfindungsgemäßen homöopathischen Mittels sowie dessen Verwendung zur Verhinderung von Infektionskrankheiten, die durch Parasiten hervorgerufen werden, sowie allgemein dessen Verwendung zur Verhinderung von Parasiten- und Insektenstichen bzw. von Tierbissen. Darüber hinaus erstreckt sich die vorliegende Erfindung auch auf Bekleidungsstücke, Schmuckstücke, Tierhalsbänder, Kristalle oder Dekorationselemente, enthaltend das erfindungsgemäße homöopathische Mittel.

Parasiten, wie beispielsweise Flöhe, Mücken, Zecken, Spinnen, Würmer, Bienen, Wespen oder Hornissen können bei Menschen und Tieren gefährliche Infektionskrankheiten hervorrufen. Dabei können diese Krankheiten zum einen dadurch ausgelöst werden, dass derartige Parasiten durch einen Biss ihr eigenes Gift auf andere Lebewesen übertragen. Es ist aber auch möglich, dass diese durch einen Biss Viren, Bakterien oder andere Erreger in andere Lebewesen einschleusen, wo diese dann gefährliche Infektionskrankheiten hervorrufen können. In diesem Fall sind solche Parasiten lediglich sogenannte Zwischenwirte für die Erreger u. a der Malaria, der Borreliose oder der Meningoenzephalitis.

Mittel und Vorrichtungen zur Vermeidung des in Kontakttretens mit solchen Parasiten und damit zur Prophylaxe gegen Infektionskrankheiten sind schon seit längerem bekannt. Die US-A 5,038,718 und die US-A 5,155,950 beschreiben jeweils Gürtel als Barriere gegenüber Zecken, die von Menschen getragen diese vor Bissen dieser Tiere schützen sollen. Derartige Gürtel enthalten u. a. klebende Oberflächen, chemisch behandelte Oberflächen oder elektrisch leitende Oberflächen und sind relativ aufwendig ausgestaltet. Weiterhin wird in der US-A 5,381,557 ein Band mit einer Klebefläche offenbart, mit dem u. a. Zecken, Spinnen oder andere Insekten abgewehrt werden können. Der relativ komplex gestaltete Gürtel weist ebenfalls adhäsiv wirkende Oberflächen auf.

Gegenstand der US-A 6,141,802 sind röhrenförmige Gebilde zur Abwehr von Zecken, die vom Benutzer innerhalb von Hosen oder Socken getragen werden sollen und zu diesem Zweck mit zeckenabweisenden Chemikalien behandelt sind. Thorsell berichtet in Fauna och Flora Naturhistoriska Riksmusect 83 (5), 202-207 über die Herstellung von Extrakten aus Ledum pallustre (marsh tea) mit Insekten abwersenden Eigenschaften.

Die vorgenannten Möglichkeiten zur Abwehr insbesondere von Zecken und Spinnen basieren alle auf relativ aufwendig ausgestalteten Bekleidungsstücken und enthalten im Regelfall Chemikalien zur Abwehr derartiger Parasiten sowie chemische Haftmittel. Bei vielen Verbrauchern besteht aber der Wunsch nach einem möglichst wirksamen aber zugleich sanften und schonenden Mittel gegen derartige Parasiten, das ohne großen Aufwand eingesetzt werden kann.

Der vorliegenden Erfindung lag daher die Aufgabe zu Grunde, den geschilderten Nachteilen abzuhelfen und ein möglichst einfach zu handhabendes Mittel zur Abwehr der genannten Parasiten, bzw. von Insekten oder Tieren allgemein, bereitzustellen, das sanft, schonend und ohne Nebenwirkungen diese Aufgabe erfüllt.

Demgemäß wurde ein homöopathisches Mittel, bzw. eine Wirkstoffkombination entwickelt, enthaltend
a) Ledum Pallustre in Form einer homöopathischen Verdünnung in einem inerten Trägermaterial und
b) wenigstens ein Sekret eines Parasiten oder eines durch Parasiten Erkrankten oder auch eines Zwischenwirts in Form einer homöopathischen Verdünnung in einem inerten Trägermaterial.

Bei dem im erfindungsgemäßen homöopathischen Mittel eingesetzten Ledum Pallustre, auch Sumpfporst oder wilder Rosmarin genannt, handelt es sich um ein in der homöopathischen Medizin eingesetztes Mittel, das u. a. durch seine antiseptischen Eigenschaften bekannt ist. Es wird in einer homöopathischen Verdünnung nach der Dezimalskala von D10 bis D1 000 000, bevorzugt von D10 bis D10 000, besonders bevorzugt von D15 bis D5000 und ganz besonders bevorzugt von D20 bis D2000 eingesetzt. Nach der Centesimalskala beträgt die homöopathische Verdünnung C1 bis C1 000 000, bevorzugt C10 bis C10 000, besonders bevorzugt C15 bis C5000 und ganz besonders bevorzugt C20 bis C2000. Ledum Pallustre (a) ist in unterschiedlichen Verdünnungen im homöopathischen Fachhandel erhältlich.

Homöopathische Verdünnungen werden im allgemeinen nach der Dezimal- oder der Centesimalskala zubereitet. Dabei werden die sogenannten Grundstoffe wie zum Beispiel Pflanzen, Bestandteile oder Sekrete von Tieren, Metallen oder Mineralien zunächst zerkleinert und dann in Wasser und/oder in Alkohol gelöst. Einige unlösliche Substanzen beispielsweise Metalle werden häufig pulverisiert, anschließend mit Milchzuckerpulver vermischt und so in eine alkohollösliche Form gebracht. Der auf diese Weise erhaltene Grundstoff wird danach in einem Wasser/Alkohol-Gemisch in einem bestimmten Verhältnis gelöst. Dieses Mischungsverhältnis hängt meistens vom Löslichkeitsverhalten der jeweiligen Substanzen ab. Die so erhaltene Mischung wird dann für eine gewisse Zeit, beispielsweise einige Stunden bis zu einigen Monaten zum Quellen gebracht. Aus dieser Mischung gewinnt man u. a. durch Extrahieren, Sieben, Filtern, Abgießen, Dekantieren oder Pressen die sogenannte "Urtinktur". Diese wiederum wird danach in einer Mischung aus Alkohol und Wasser so lange gelöst, bis die gewünschte Potenz vorliegt. Bei dem sogenannten Potenzieren werden die homöopathischen Verdünnungen wie folgt hergestellt.

Um eine D1-Verdünnung (Dezimal 1) zu erzielen, versetzt man 1 Tropfen der jeweiligen Urtinktur mit 9 Tropfen einer Lösung von üblicherweise destilliertem Wasser und Alkohol (Ethanol). Der Alkoholgehalt dieser Lösung wiederum hängt entscheidend vom Löslichkeitsverhalten der verwendeten Substanzen ab und liegt meist im Bereich von 5 bis 95 Gew.-%, insbesondere im Bereich von 10 bis 90 Gew.-%. Die so erhaltene Lösung wird dann mehrmals verschüttelt (geschüttelt). Auf diese Weise erhält man eine sogenannte D1-Verdünnung. Werden 1 Tropfen dieser D1-Verdünnung mit 9 Tropfen einer alkoholischen Wasserlösung (im gleichen Mischungsverhältnis wie für D1-Verdünnungen) vereinigt und anschließend mehrfach verschüttelt (geschüttelt), so resultiert daraus eine sogenannte D2-Verdünnung. Zu einer D3-Verdünnung gelangt man dadurch, dass man einer D2-Verdünnung einen Tropfen entnimmt, diesen in 9 Tropfen einer alkoholischen Wasserlösung auflöst und dann mehrmals verschüttelt. Bei einer D20-Verdünnung wird dieser Vorgang ausgehend von der Urtinktur insgesamt 20 Mal durchgeführt.

Bei einer sogenannten C1-Verdünnung (Centesimal 1) entnimmt man einen Tropfen der sogenannten Urtinktur und gibt ihn in 99 Tropfen einer Wasser/Alkohol-Mischung, die danach mehrfach verschüttelt wird. Aus einem Tropfen dieser C1-Verdünnung und 99 Tropfen einer alkoholischen Wasserlösung erhält man durch entsprechendes Verschütteln dann eine sogenannte C2-Verdünnung. Dieser Vorgang ist immer so lange zu wiederholen, bis die gewünschte Verdünnung erreicht ist, beispielsweise bei einer C200 Verdünnung also insgesamt 200 Mal.

Die auf diese Weise gewonnene homöopathische Verdünnung wird entweder direkt als sogenannte Tropfenform dargereicht oder aber mit einem festen inerten Trägermaterial, beispielsweise mit Milchzucker, Stärke oder anderen Zuckerarten vermengt und in Form von Globuli (Kügelchen) oder aber in Form von Tabletten als homöopathische Arznei angeboten. Im Falle von homöopathischen Tropfen handelt es sich bei dem inerten Trägermaterial üblicherweise um Lösungen aus Alkohol und Wasser.

Weiterhin enthält das erfindungsgemäße homöopathische Mittel wenigstens ein Sekret (b) eines Parasiten und/oder eines durch Parasiten Erkrankten in Form einer homöopathischen Verdünnung in einem inerten Trägermaterial, beispielsweise Wasser und/oder Alkohol oder Milchzucker, Stärke oder andere Zuckerarten. Bei dem Sekret (b) eines durch Parasiten Erkrankten (Mensch oder Tier) handelt es sich um verschiedenartige, ausgeschiedene Körperflüssigkeiten oder feste Körperbestandteile eines üblicherweise durch einen Biss (Flöhe, Spinnen oder Zecken), durch einen Stich (Bienen, Wespen, Hummeln oder Hornissen) oder durch einen sonstigen Kontakt (Würmer) Infizierten. Dieses Sekret wird in einem Gemisch aus Wasser und Alkohol gelöst und nach den Regeln der Homöopathie durch fortwährendes Lösen und Verschütteln entsprechend potenziert. Bevorzugt enthält der Bestandteil (b) u. a. ein Sekret eines Borrellioseerkrankten und/oder eines Meningoenzephalitiserkrankten (sowohl der in Europa, als auch der in Nordamerika bzw. in anderen Regionen der Erde vorkommenden Erreger) und/oder das Sekret eines an einem Mückenstich oder an einer anderen durch Bisse oder Stiche übertragenen Krankheit Erkrankten.

Weiterhin bevorzugt ist ein solcher Bestandteil (b), der ein Sekret der die Malaria übertragenden Anopheles-Mücke aufweist oder der ein Sekret eines an der Malaria Erkrankten enthält. Bei der Malaria handelt es sich um eine Sammelbezeichnung für fiebrige Infektionen durch Protozoen (tierische Einzeller), die durch den Stich der Anopheles-Mücke übertragen werden. Das erfindungsgemäße homöopathische Mittel kann dabei ein oder mehrere verschiedene Sekrete der drei üblicherweise vorkommenden Malaria-Formen aufweisen, d.h. der Malaria tertiana (Dreitagefieber), der Malaria quartana sowie der Malaria tropica.

Das Sekret b) ist in einer homöopathischen Verdünnung nach der Dezimalskala von D1 bis D1 000 000, vorzugweise von D10 bis D10 000, besonders bevorzugt von D15 bis D5 000 und ganz besonders bevorzugt von D20 bis D 2000 einzusetzen. Nach der Centesimalskala beträgt die homöopathische Verdünnung C1 bis C1 000 000, bevorzugt C10 bis C10 000, besonders bevorzugt C15 bis C 5000 und ganz besonders bevorzugt C20 bis C2 000. Das Sekret b) wird entweder direkt als sogenannte Tropfenform mit Wasser und/oder Alkohol als inerte Trägermaterialien eingesetzt oder aber auf inerte, feste Trägermaterialien wie Milchzucker, Stärke oder andere Zuckerarten aufgebracht und in Form von Globuli oder Tabletten dargereicht.

Es ist in dieser Form im homöopathischen Fachhandel käuflich.

Darüber hinaus kann das erfindungsgemäße homöopathische Mittel noch Delphinium staphysagria c) in Form einer homöopathischen Verdünnung in einem inerten Träger-material enthalten. Es wird c) Delphinium staphysagria (auch Rittersporn genannt) in einer homöopathischen Verdünnung nach der Dezimalskala von D1 bis D1 000 000, insbesondere von D10 bis D10 000, besonders bevorzugt von D15 bis D5000 und ganz besonders bevorzugt von D20 bis D2 000 eingesetzt. Nach der Centesimalskala beträgt die homöopathische Verdünnung für Delphinium staphysagria c) C1 bis C1 000 000, insbesondere C10 bis C10 000, besonders bevorzugt C15 bis C5000 und ganz besonders bevorzugt C20 bis C2000. Geeignete Trägermaterialien sind auch hier u. a. Wasser und/oder Alkohol (falls die Darreichungsform als Tropfen vorliegt) oder aber Milchzucker, Stärke und andere Zuckerarten (falls c) in Form von Globuli oder Tabletten eingesetzt wird). Delphinium staphysagria c) ist in den gewünschten homöopathischen Verdünnungen im Fachhandel erhältlich.

Nach dem ebenfalls erfindungsgemäßen Verfahren zur Herstellung der erfindungsgemäßen homöopathischen Mittel werden die durch Verdünnen und durch Verschütteln gewonnenen homöopathischen Mittel a), b) sowie gegebenenfalls c) zunächst auf ein inertes Trägermaterial gebracht (Wasser und/oder Alkohol bzw. inerte feste Trägermaterialien wie Milchzucker, Stärke oder andere Zuckerarten) und anschließend als Mischung in Form von verschiedenen Globuli, Tabletten oder als Lösung in einem geeigneten Behälter oder auf einem geeigneten Stoff aufbewahrt. Das Aufbringen der erfindungsgemäßen homöopathischen Mittel auf feste inerte Trägermaterialien erfolgt üblicherweise durch Auftropfen der in der gewünschten Potenz vorliegenden Lösung auf die Trägermaterialien.

Die einzelnen Substanzen des erfindungsgemäßen homöopathischen Mittels, also a) und b), sowie gegebenenfalls c) liegen dabei vorzugsweise in solchen Mengenverhältnissen vor, dass auf Ledum Pallustre a) ein Mengenanteil von 10 bis 90 Gewichtsprozent, insbesondere von 15 bis 80 Gewichtsprozent und ganz besonders bevorzugt von 20 bis 70 Gewichtsprozent fällt. Das Sekret b) liegt entsprechend in einem Mengenverhältnis von 10 bis 90 Gewichtsprozent, insbesondere von 15 bis 80 Gewichtsprozent und besonders bevorzugt von 20 bis 70 Gewichtsprozent vor, wobei sich a) und b) zu jeweils 100 Gewichtsprozent addieren.

Falls im homöopathischen Mittel neben a) und b) noch Delphinium staphysagria c) vorhanden ist, liegen die Mengenanteile von a), b) und c) jeweils bei 10 bis 80 Gewichtsprozent, insbesondere bei 15 bis 70 Gewichtsprozent und ganz besonders bevorzugt bei 20 bis 60 Gewichtsprozent, wobei sich die Mengenanteile von a), b) und c) jeweils auf 100 Gewichtsprozent summieren. Vorzugsweise liegen die Substanzen a), b) und gegebenenfalls c) in jeweils etwa gleichen Mengenanteilen vor.

Das erfindungsgemäße homöopathische Mittel wird insbesondere in Form einer Lösung der Wirkstoffe a), b) sowie gegebenenfalls c) oder in Form von verschiedenen Globuli oder Tabletten, die jeweils einen Wirkstoff a), sowie b) und gegebenenfalls c) aufweisen, in einem geeigneten Behälter oder auf einem geeigneten Stoff aufbewahrt.

Es kann sich empfehlen, das homöopathische Mittel in ein Bekleidungsstück, beispielsweise in einen Gürtel, einen Beutel, ein Band oder in ein sonstiges am Körper zu tragendes Aufbewahrungsbehältnis zu verstauen. Dies kann selbstverständlich auch ein Schmuckstück sein, beispielsweise ein Ring, ein Halsband, eine Kette oder aber eine Brosche, in dem das homöopathische Mittel aufbewahrt, bzw. äußerlich aufgebracht und versiegelt wird.

Darüber hinaus ist es auch möglich, das erfindungsgemäße homöopathische Mittel allgemein auf ein Trägermaterial oder Subsrat direkt aufzubringen, insbesondere auf einen Kristall oder ein Dekorationselement wie beispielsweise einen Schmuckstein; d.h. das erfindungsgemäße Mittel nicht in einem Behälter aufzubewahren. Dabei wird die energetische Struktur des erfindungsgemäßen homöopathischen Mittels auf den Kristall oder auf das Dekorationselement übertragen.

Das erfindungsgemäße homöopathische Mittel eignet sich insbesondere zur Vermeidung von Bissen und Stichen von Parasiten, Insekten und anderen Tieren und von Infektionskrankheiten, die durch Parasiten hervorgerufen werden, beispielsweise zur Verhinderung der Borrelliose, der Meningoenzephalitis, also von durch Zecken übertragenen Krankheiten, sowie von Krankheiten, die auf Mückenstiche bzw. Fliegenstiche zurück zuführen sind, wie beispielsweise Malaria oder die Schlafkrankheit (afrikanische Trypanosomiasis).

Dabei wird die entsprechende Wirkung bereits dadurch ausgelöst, dass das homöopathische Mittel am Körper getragen wird, ohne dass es dabei nötig ist, dieses oral einzunehmen. Es eignet sich daher vor allem als sogenanntes prophylaktisch wirksames Mittel zur Verhinderung von Bissen oder Stichen von Parasiten, insbesondere von Zecken oder Mücken und damit zur Verhinderung entsprechend übertragener Infektionskrankheiten.

Die Wirkungsweise des erfindungsgemäßen homöopathischen Mittels soll an Hand der nachfolgenden Beispiele näher erläutert werden.

### Beispiele

Es wurden insgesamt 5 Globuli Ledum Pallustre a) in der homöopathischen VerdünnungC200 aufgebracht auf Milchzucker, mit 5 Globuli Borrellia b) (aus dem Sekret eines an Borrelliose Erkrankten) in der Verdünnung C200 und aufgebracht auf Milchzucker, sowie mit 5 Globuli FSME b) (aus dem Sekret eines an Frühsommerenzephalitis Erkrankten) in der Verdünnung C200 und aufgebracht auf Milchzucker und ferner mit 5 Globuli Staphysagria c) in der Verdünnung C 200 und aufgebracht auf Milchzucker in einem Beutel vereinigt. Da jedes der Globuli ca. 50 mg wog, ergab sich ein Gesamtgewicht von ca. 1000 mg für die insgesamt 20 Globuli.

Die dabei eingesetzten Globuli wurden durch entsprechendes Potenzieren bis auf C200 und Verschütteln mit Hilfe eines Gemisches aus Alkohol und Wasser nach dem deutschen Arzneimittelhandbuch gewonnen.

Analog dazu wurden die gleichen Substanzen a) (Verdünnung : C200), b) (Borrellia, Verdünnung : C200) und b)(FSME, Frühsommerenzephalitis, Verdünnung : C200), sowie c) (Verdünnung : C200) in Form von 4 Tabletten mit einem Einzelgewicht von jeweils 260 mg in einem kleinen Behälter vereinigt.

Mit diesen homöopathischen Mitteln wurden folgende Versuche durchgeführt:

### Beispiel 1 :

In einem Langzeitversuch wurde das erfindungsgemäße homöopathische Mittel über mehrere Jahre hinweg zur Zeckenprophylaxe täglich am Körper getragen. Dabei wurde durch das bloße Tragen des Mittels die Zahl der Zeckenbisse um ca. 70 bis 98 % verringert.

### Beispiel 2 :

Weiterhin wurde einem Jäger, der jedes Jahr ca. 20 - 30 Zeckenbissen ausgesetzt war, das homöopathische Mittel als Beutel verabreicht, den er täglich in seiner Hosentasche trug. Seitdem wurde bei ihm kein einziger Zeckenbiss mehr beobachtet.

### Beispiel 3 :

Einem Landwirt, der in Folge eines Zeckenbisses schwer an Borreliose erkrankt war und unter starken Gelenkschmerzen litt, wurde das erfindungsgemäße homöopathische Mittel zum äußerlichen Tragen verabreicht. Seitdem er dieses ständig bei sich trägt, wurde er nicht mehr von Zecken gebissen, mit Ausnahme auf einer Fahrradtour, bei der er die o. g. Wirkstoffkombination nicht bei sich trug.

### Beispiel 4 :

Einem Schäferhund, der seit seiner Welpenzeit immer zahlreiche Zeckenbisse hatte, wurde das erfindungsgemäße homöopathische Mittel an seinem Halsband befestigt. Seit dieser Zeit ist er praktisch zeckenfrei.

### Beispiel 5 :

Ein weiterer Patient, der gerne im Garten arbeitet, verwendet seit einiger Zeit das erfindungsgemäße homöopathische Mittel und ist seither von Zeckenbissen verschont geblieben, ausgenommen wenn er vergaß, das erfindungsgemäße Mittel in der Tasche bei sich zu tragen.

Die Beispiele 2 - 4 beweisen eindeutig, dass die Wirksamkeit des erfindungsgemäßen homöopathischen Mittels nicht auf einem Placeboeffekt beruht, d.h. nicht durch eine bloße Einbildung des Patienten hervorgerufen wird.

## Patentansprüche

1. Homöopathisches Mittel, enthaltend,
a) Ledum Pallustre in Form einer homöopathischen Verdünnung in einem inerten Trägermaterial und
b) wenigstens ein Sekret eines Parasiten oder eines durch Parasiten Erkrankten oder eines Zwischenwirts in Form einer homöopathischen Verdünnung in einem inerten Trägermaterial, **dadurch gekennzeichnet, dass** das Ledum Pallustre in einer homöopathischen Verdünnung nach der Dezimalskala von D10 bis D1 000 000 oder nach der Centesimalskala von C1 bis C 1 000 000 enthalten ist.

2. Homöopathisches Mittel nach Anspruch 1, enthaltend weiterhin
c) Delphinium staphysagria in Form einer homöopathischen Verdünnung in einem inerten Trägermaterial.

3. Homöopathisches Mittel nach einem der Ansprüche 1 oder 2, enthaltend
b) wenigstens ein Sekret eines Borrellioseerkrankten in Form einer homöopathischen Verdünnung in einem inerten Trägermaterial.

4. Homöopathisches Mittel nach einem der Ansprüche 1 bis 3, enthaltend
b) wenigstens ein Sekret eines Meningoenzephalitiserkrankten in Form einer homöopathischen Verdünnung in einem inerten Trägermaterial.

5. Homöopathisches Mittel nach einem der vorhergehenden Ansprüche, enthaltend
a) Ledum Pallustre in einer homöopathischen Verdünnung nach der Dezimalskala von D10 bis D10 000 oder nach der Centesimalskala von C10 bis C10 000.

6. Homöopathisches Mittel nach einem der Ansprüche 1 bis 5, enthaltend
b) wenigstens ein Sekret eines Parasiten oder eines durch Parasiten Erkrankten in einer homöopathischen Verdünnung nach der Dezimalskala von D1 bis D1 000 000 oder nach der Centesimalskala von C1 bis C1 000 000.

7. Homöopathisches Mittel nach Anspruch 6, enthaltend
b) wenigstens ein Sekret eines Parasiten oder eines durch Parasiten Erkrankten in einer homöopathischen Verdünnung nach der Dezimalskala von D10 bis D10 000 oder nach der Centesimalskala von C10 bis C10 000.

8. Homöopathisches Mittel nach einem der Ansprüche 2 bis 7, enthaltend c)Delphinium staphysagria in einer homöopathischen Verdünnung nach der Dezimalskala von D1 bis D1 000 000 oder nach der Centesimalskala von C1 bis C1 000 000.

9. Homöopathisches Mittel nach Anspruch 8, enthaltend
c) Delphinium staphysagria in einer homöopathischen Verdünnung nach der Dezimalskala von D10 bis D10 000 oder nach der Centesimalskala von C10 bis C10 000.

10. Homöopathische Mittel nach einem der Ansprüche 1 bis 9, enthaltend als inerte Trägermaterialien Milchzucker für Globuli oder für Tabletten oder Wasser bzw. Gemische aus Wasser und Alkohol für Tropfen.

11. Verfahren zur Herstellung eines homöopathischen Mittels gemäß einem der Ansprüche 1 bis 10, wobei die durch Verdünnen und durch Verschütteln gewonnenen homöopathischen Mittel a), b) sowie gegebenenfalls c) zunächst auf ein inertes Trägermaterial aufgebracht werden und anschließend als Mischung in Form von verschiedenen Globuli, Tabletten oder als Lösung in einem geeigneten Behälter oder auf einem geeigneten Stoff aufbewahrt werden.

12. Substrat, insbesondere Bekleidungsstück, Schmuckstück oder Tierhalsband, enthaltend ein homöopathisches Mittel nach einem der Ansprüche 1 bis 10 oder eine dem homöopathischen Mittel entsprechende energetische Struktur.

13. Kristalle oder Dekorationselemente, enthaltend ein homöopathisches Mittel nach einem der Ansprüche 1 bis 10.

14. Verwendung eines ankörper getragenen homöopathischen Mittels gemäß einem der Ansprüche 1 bis 10 zur Verhinderung von Bissen und/oder Stichen durch Parasiten, die Infektionskrankheiten hervorrufen können, oder die Erreger übertragen, welche Infektionskrankheiten hervorrufen können.

15. Verwendung eines am Körper getragenen homöopathischen Mittels gemäß Anspruch 14 zur Verhinderung von Infektionskrankheiten, die durch den Biss von Zecken oder den Stich von Mücken hervorgerufen werden.

## Claims

1. Homeopathic agent, containing
a) Ledum palustre in a form of a homeopathic dilution in an inert carrier material, and
b) at least one secretion from a parasite, a sufferer of a disease caused by parasites, or an intermediate host, in the form of a homeopathic dilution in an inert carrier material, **characterised in that** the Ledum palustre is contained in a homeopathic dilution of D10 to D 1,000,000 on the decimal scale or of C1 to C1,000,000 on the centesimal scale.

2. Homeopathic agent according to claim 1, further containing
c) Delphinium staphysagria in the form of a homeopathic dilution in an inert carrier material.

3. Homeopathic agent according to either claim 1 or claim 2, containing
b) at least one secretion from a sufferer of borreliosis, in the form of a homeopathic dilution in an inert carrier material.

4. Homeopathic agent according to any one of claims 1 to 3, containing
b) at least one secretion from a sufferer of meningoencephalitis, in the form of the homeopathic dilution in an inert carrier material.

5. Homeopathic agent according to any one of the preceding claims, containing
b) Ledum palustre in a homeopathic dilution of D 10 to D 10,000 on the decimal scale or of C10 to C 10,000 on the centesimal scale.

6. Homeopathic agent according to any one of claims 1 to 5, containing
b) at least one secretion from a parasite or a sufferer of a disease caused by parasites, in a homeopathic dilution of D1 to D1,000,000 on the decimal scale or of C1 to C 1,000,000 on the centesimal scale.

7. Homeopathic agent according to claim 6, containing
b) at least one secretion from a parasite or a sufferer of a disease caused by parasites, in a homeopathic dilution of D10 to D 10,000 on the decimal scale or of C10 to C 10,000 on the centesimal scale.

8. Homeopathic agent according to any one of claims 2 to 7, containing
c) Delphinium staphysagria in a homeopathic dilution of D1 to D 1,000,000 on the decimal scale or of C1 to C 1,000,000 on the centesimal scale.

9. Homeopathic agent according to claim 8, containing
c) Delphinium staphysagria in a homeopathic dilution of D10 to D 10,000 on the decimal scale or of C 10 to C 10,000 on the centesimal scale.

10. Homeopathic agent according to any one of claims 1 to 9, containing lactic sugar, for globules or tablets, or water or mixtures of water and alcohol, for drops, as the inert carrier material.

11. Method for producing a homeopathic agent according to any one of claims 1 to 10, wherein the homeopathic agents a), b) and optionally c), which are obtained by dilution and succession, are initially applied to an inert carrier material and subsequently stored as a mixture in the form of various globules or tablets, or as a solution in a suitable container or on a suitable material.

12. Substrate, in particular an item of clothing, item of jewellery or animal collar, containing a homeopathic agent according to any one of claims 1 to 10 or an energetic structure corresponding to the homeopathic agent.

13. Crystals or decorative elements containing a homeopathic agent according to any one of claims 1 to 10.

14. Use of a homeopathic agent, worn on the body, according to any one of claims 1 to 10, for the prevention of bites and/or stings by parasites, which can bring about infectious diseases or can transmit the pathogens which can bring about infectious diseases.

15. Use of a homeopathic agent, worn on the body, according to claim 14, for the prevention of infectious diseases which are brought about by tick bites or mosquito bites.

## Revendications

1. Agent homéopathique contenant
a) Ledum Pallustre sous la forme d'une dilution homéopathique dans une matière de support inerte, et
b) au moins une sécrétion d'un parasite ou d'un patient atteint d'une maladie parasitaire ou d'un hôte intermédiaire, sous la forme d'une dilution homéopathique dans une matière de support inerte, **caractérisé en ce que** le Ledum Pallustre est contenu dans une dilution homéopathique conformément à l'échelle décimale de D10 à D1.000.000 ou conformément à l'échelle centésimale de C1 à C1.000.000.

2. Agent homéopathique selon la revendication 1, contenant en outre
c) Delphinium staphysagria sous la forme d'une dilution homéopathique dans une matière de support inerte.

3. Agent homéopathique selon l'une quelconque des revendications 1 ou 2, contenant
b) au moins une sécrétion d'un patient atteint de borréliose, sous la forme d'une dilution homéopathique dans une matière de support inerte.

4. Agent homéopathique selon l'une quelconque des revendications 1 à 3, contenant
b) au moins une sécrétion d'un patient atteint d'une méningo-encéphalite sous la forme d'une dilution homéopathique dans une matière de support inerte.

5. Agent homéopathique selon l'une quelconque des revendications précédentes, contenant
a) Ledum Pallustre dans une dilution homéopathique conformément à l'échelle décimale de D10 à D10.000 ou conformément à l'échelle centésimale de C10 à C10.000.

6. Agent homéopathique selon l'une quelconque des revendications 1 à 5, contenant
b) au moins une sécrétion d'un parasite ou d'un patient atteint d'une maladie parasitaire dans une dilution homéopathique conformément à l'échelle décimale de D1 à D1.000.000 ou conformément à l'échelle centésimale de C1 à C1.000.000.

7. Agent homéopathique selon la revendication 6, contenant
b) au moins une sécrétion d'un parasite ou d'un patient atteint d'une maladie parasitaire dans une dilution homéopathique conformément à l'échelle décimale de D10 à D10.000 ou conformément à l'échelle centésimale de C10 à C10.000.

8. Agent homéopathique selon l'une quelconque des revendications 2 à 7, contenant
c) Delphinium staphysagria dans une dilution homéopathique conformément à l'échelle décimale de D1 à D1.000.000 ou conformément à l'échelle centésimale de C1 à C1.000.000.

9. Agent homéopathique selon la revendication 8, contenant
c) Delphinium staphysagria dans une dilution homéopathique conformément à l'échelle décimale de D10 à D10.000 ou conformément à l'échelle centésimale de C10 à C10.000.

10. Agent homéopathique selon l'une quelconque des revendications 1 à 9, contenant, à titre de matières de support inertes, du lactose pour des globules ou pour des comprimés ou bien de l'eau respectivement des mélanges d'eau et d'alcool pour des gouttes.

11. Procédé pour la préparation d'un agent homéopathique selon l'une quelconque des revendications 1 à 10, dans lequel, dans un premier temps, on applique sur une matière de support inerte les agents homéopathiques a) b) et le cas échéant c), obtenus par dilution et par agitation, et ensuite on les entrepose sous forme d'un mélange sous la forme de différents globules, de différents comprimés ou sous la forme d'une solution dans un récipient approprié ou encore sur une substance appropriée.

12. Substrat, en particulier vêtement, bijou ou collier pour animal, contenant un agent homéopathique selon l'une quelconque des revendications 1 à 10, ou une structure énergétique correspondant à l'agent homéopathique.

13. Cristaux ou éléments de décoration, contenant un agent homéopathique selon l'une quelconque des revendications 1 à 10.

14. Utilisation d'un agent homéopathique porté sur le corps, selon l'une quelconque des revendications 1 à 10, pour empêcher les morsures et/ou les piqûres dues à des parasites, qui peuvent être à l'origine de maladies infectieuses ou qui transmettent des agents pathogènes qui peuvent être à l'origine de maladies infectieuses.

15. Utilisation d'un agent homéopathique porté sur le corps selon la revendication 14, pour empêcher des maladies infectieuses qui sont provoquées par la morsure de tiques ou par la piqûre de moustiques.
